(19) Europäisches Patentamt
European Patent Office
Office européen des brevets

(11) **EP 3 010 569 B1**

(12) **EUROPÄISCHE PATENTSCHRIFT**

(45) Veröffentlichungstag und Bekanntmachung des Hinweises auf die Patenterteilung:
**22.04.2020   Patentblatt 2020/17**

(21) Anmeldenummer: **14730929.8**

(22) Anmeldetag: **18.06.2014**

(51) Int Cl.:
*A61M 5/44* (2006.01)

(86) Internationale Anmeldenummer:
**PCT/EP2014/062838**

(87) Internationale Veröffentlichungsnummer:
**WO 2014/202674 (24.12.2014 Gazette 2014/52)**

(54) **MEDIZINISCHER WÄRMEÜBERTRAGER ZUM ERWÄRMEN MEDIZINISCHER FLÜSSIGKEITEN MITTELS EINES EMITTERS FÜR LICHT UND MEDIZINISCHE FLÜSSIGKEITSBEHANDLUNGSVORRICHTUNG MIT EINEM EMITTER FÜR LICHT**

MEDICAL HEAT EXCHANGER FOR HEATING MEDICAL LIQUIDS BY MEANS OF AN EMITTER FOR LIGHT AND MEDICAL LIQUID TREATMENT DEVICE HAVING AN EMITTER FOR LIGHT

ÉCHANGEUR THERMIQUE MÉDICAL POUR RÉCHAUFFER DES LIQUIDES MÉDICAUX AU MOYEN D'UN ÉMETTEUR POUR LUMIÈRE ET DISPOSITIF DE TRAITEMENT DE LIQUIDE MÉDICAL COMPRENANT UN ÉMETTEUR POUR LUMIÈRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.06.2013   DE 102013010106**

(43) Veröffentlichungstag der Anmeldung:
**27.04.2016   Patentblatt 2016/17**

(73) Patentinhaber: **Fresenius Medical Care Deutschland GmbH**
**61352 Bad Homburg (DE)**

(72) Erfinder:
• **LAUER, Martin**
**66606 St. Wendel (DE)**
• **PETERS, Arne**
**61352 Bad Homburg (DE)**

(74) Vertreter: **Dreyhsig, Jörg et al**
**Fresenius Medical Care AG & Co. KGaA**
**Global Intellectual Property**
**Frankfurter Strasse 6-8**
**66606 St. Wendel (DE)**

(56) Entgegenhaltungen:
EP-A2- 2 311 514          DE-A1-102010 002 895
US-A1- 2003 135 250     US-A1- 2009 299 271

**Beschreibung**

**Technisches Gebiet**

Die Erfindung betrifft einen medizinischen Wärmeübertrager zum Erwärmen medizinischer Flüssigkeiten mittels eines Emitters für Licht und eine medizinische Flüssigkeitsbehandlungsvorrichtung mit einem Emitter für Licht.

Es gibt eine Vielzahl von Aufgabenstellungen in der Medizintechnik, bei denen eine medizinische Flüssigkeit erwärmt werden soll, beispielsweise um eine Abkühlung des Patienten durch die medizinische Flüssigkeit zu vermeiden und/ oder um eine vorgegebene Temperatur der medizinischen Flüssigkeit nahe der Körpertemperatur des Patienten einzustellen. Beispiele für solche medizinische Aufgabenstellung sind die Erwärmung von Blut in einem extrakorporalen Blutkreislauf oder die Erwärmung von Dialysierflüssigkeit in einem Dialysierflüssigkeitskreislauf oder die Erwärmung von Substitutionsflüssigkeit, wie dies bei der Hämodialyse, Hämofiltration und Hämodiafiltration vorkommt. Bei Blutbehandlungsverfahren mit extrakorporalem Blutkreislauf wird dem Patienten über eine arterielle Dialysenadel aus einem als Fistel oder Shunt ausgeführten vaskulären Zugang Blut entnommen, das von der arteriellen Dialysenadel über eine arterielle Schlauchleitung beispielsweise mittels einer Blutpumpe zu einer Blutbehandlungseinheit, z.B. einem Dialysator oder Filter abgeführt wird und nach Durchströmen der Blutbehandlungseinheit über eine venöse Schlauchleitung zurück in die Fistel oder den Shunt des Patienten geleitet wird. Das Blut kann sich im extrakorporalen Blutkreislauf durch Wärmeverluste an die Umgebung merklich abkühlen. Mittels eines medizinischen Wärmeübertragers kann das Blut vor der Rückgabe in den Patienten auf eine vorgegebene Temperatur erwärmt werden. Ein dazu geeigneter medizinischen Wärmeübertrager kann in oder an der venösen Schlauchleitung angeordnet sein. Es sind medizinische Wärmeübertrager bekannt, bei denen ein Leitungsabschnitt der venösen Leitung um eine elektrische Heiztrommel gewickelt wird.

Weitere Beispiele für die Anwendung eines medizinischen Wärmeübertragers sind die Erwärmung von Dialysierflüssigkeit bei der Peritonealdialyse oder die Erwärmung von Infusionslösungen in der Infusionstechnik.

**Hintergrund**

Die Erwärmung einer medizinischen Flüssigkeit mittels eines medizinischen Wärmeübertragers kann durch elektrische Widerstands-Heizelemente wie z.B. Heizplatten oder mittels elektrischer Emitter für Licht erfolgen, die den medizinischen Wärmeübertrager indirekt von außen beheizen.

In der Schrift US 2003/0135250 A1 wird ein mit Strahlungsenergie beheizter Wärmeübertrager beschrieben zur Erwärmung eines Fluids, wie einer parenteralen Flüssigkeit oder einer Dialysierflüssigkeit für die Verwendung in einer Dialysetherapie.

In der Schrift US 2009/0299271 A1 wird ein mit einer Lichtquelle beheizter Wärmeübertrager mit einer Fluidkassette zur Ewärmung einer Flüssigkeit, beispielsweise für die Peritonealdialyse, beschrieben.

In der Schrift DE 10 2010 002895 A1 wird eine Vorrichtung zum Erwärmen von strömenden Fluiden beschrieben, mit einem Fluidgehäuse enthaltend einen mäanderförmigen oder spiralförmigen Fluidkanal und mit einer Erwärmungseinheit enthaltend mindestens ein elektrisches Flächenheizelement, wobei das elektrische Flächenheizelement in einem Arbeitszustand in einer direkten wärmeleitenden Verbindung zu dem Fluid steht.

In der Schrift EP 2 311 514 A2 wird eine Erwärmungsvorrichtung zur medizinischen Behandlung beschrieben. Die Erwärmungsvorrichtung gemäß einer Ausführungsform umfasst einen Heizer; ein Sperrelement und ein Abdeckelement, die an einer Vorderseite und einer Rückseite des Heizelements angeordnet sind und einen Fluidpfad bilden, der es ermöglicht, dass das Fluid in einer Weise bewegt wird, dass es den Heizer umgibt.

In der Schrift WO 03/061740 A1 wird ein medizinischer Wärmeübertrager beschrieben, der als Disposable Heizkassette ausgeführt ist, wobei die Beheizung der Heizkassette von außen einerseits durch einen an die Heizkassette angekoppelten elektrischen Emitter für Licht und andererseits auf der gegenüberliegenden Seite der Heizkassette zusätzlich mit einer an die Heizkassette angekoppelten elektrischen Widerstands-Heizplatte erfolgt. In einer alternativen Ausführungsform weist die elektrische Heizplatte außerhalb der Heizkassette zusätzlich einen Absorber für Infrarotstrahlung auf, der die Heizkassette zusätzlich von außen beheizt.

Die aus dem Stand der Technik bekannten gattungsgemäßen medizinischen Wärmeübertrager weisen beträchtliche Nachteile auf, weil die maschinenseitige Ankoppelfläche des Emitters für Licht und die Ankoppelfläche der Disposable Heizkassette thermisch hoch belastet sind, wobei die maximal auftretenden Temperaturen sowohl aufgrund von Sicherheitsaspekten als auch aufgrund von Werkstoffaspekten beispielsweise 80 °C nicht überschreiten dürfen. Deshalb werden zumindest große Ankoppelflächen und eine mechanisch aufwendige Ankopplung der Disposable Heizkassette an die maschinenseitige Ankoppelfläche des Emitters für Licht benötigt. Außerdem kann zusätzlich die Absaugung der beim Ankoppeln zwischen der Ankoppelfläche der Disposable Heizkassette und der maschinenseitigen Ankoppelfläche des Emitters für Licht eingeschlossenen Luft erforderlich sein. Wenn die Ankoppelfläche der Disposable Heizkassette als Folie ausgebildet ist, so kann in Verbindung mit der Absaugung der Luft auch eine aufwendige Anpressung der Folie an die Ankoppelfläche des Emitters für Licht erforderlich sein. Trotz solcher aufwendiger Maßnahmen muss dennoch ein beträchtlicher Teil der Strahlung des Emitters für Licht durch maschinenseitige Bauteilkühlung abgeführt werden und steht nicht für die Erwärmung der medizinischen Flüssigkeit zur Verfügung.

Beim Durchgang von Licht durch Materie wird ein Teil des Lichts in Wärme umgewandelt und ein Teil des Lichts wird durchgelassen.

Innerhalb einer sehr kleinen Schichtdicke dx der Materie wird die Strahlungsintensität des Lichts um den gleichen Bruchteil verringert, was durch den Absorptionskoeffizienten beschrieben wird. Der Absorptionskoeffizient ist eine stoffspezifische Funktion in Abhängigkeit von der Wellenlänge $\lambda$ des Lichts, die als Absorptionsspektrum $\mu(\lambda)$ des Stoffs bezeichnet wird. Je größer der Absorptionskoeffizient bei einer bestimmten Wellenlänge ist, desto größer ist die in der sehr kleinen Schicht dx absorbierte Strahlungsintensität des Lichts einer bestimmten Wellenlänge, die in der Schicht in Wärme umgewandelt wird.

Analog beschreibt der Transmissionskoeffizient den Durchgang des Lichts durch eine sehr kleine Schichtdicke dx der Materie. Der Transmissionskoeffizient ist in analoger Weise eine spezifische Funktion der Wellenlänge $\lambda$ des Lichts, die als Transmissionsspektrum $\mu(\lambda)$ des Stoffs bezeichnet wird. Je größer der Transmissionskoeffizient bei einer bestimmten Wellenlänge ist, desto größer ist die durch die sehr kleine Schicht dx durchgelassene Strahlungsintensität des Lichts der bestimmten Wellenlänge.

Der Absorptionsgrad $\alpha$ eines Körpers ist definiert als der Quotient aus absorbierter Strahlungsenergie und auftreffender Strahlungsenergie. Der Absorptionsgrad ist daher ein Maß zur Charakterisierung eines Körpers hinsichtlich seiner Eigenschaft, auftreffendes Licht in Wärme umzuwandeln.

Der Transmissionsgrad $\tau$ eines Körpers ist definiert als der Quotient aus durchgelassener Strahlungsenergie und auftreffender Strahlungsenergie.

Körper, die alle auftreffende Strahlung absorbieren und vollständig in Wärme umwandeln, weisen den Absorptionsgrad 1 und den Transmissionsgrad 0 auf ("absolut schwarze Körper"). Der Reflexionsgrad $\rho$ eines Körpers ist definiert als der Quotient aus reflektierter Strahlungsenergie und auftreffender Strahlungsenergie.

Es gilt:

$$\text{Transmissionsgrad } \tau + \text{Absorptionsgrad } \alpha + \text{Reflexionsgrad } \rho = 1$$

Körper die alle auftreffende Strahlung reflektieren oder alle auftreffende Strahlung durchlassen, weisen den Absorptionsgrad 0 auf.

Ein bekannter Vorteil der gattungsgemäßen durch einen Emitter für Licht beheizten medizinischen Wärmeübertrager ist die hohe elektrische Sicherheit aufgrund des räumlichen Abstands zwischen dem Emitter für Licht und der Flüssigkeit.

Ein bekanntes Problem der gattungsgemäßen durch einen Emitter für Licht beheizten medizinischen Wärmeübertrager ist das zumindest lokale Auftreten von unzulässigen Temperaturspitzen, wobei das Problem insbesondere bei medizinischen Wärmeübertragern aus Werkstoffen mit geringer Wärmeleitfähigkeit auftritt. Als Folge der geringen Wärmeleitfähigkeit geben solche medizinischen Wärmeübertrager einen beträchtlichen Teil der eingestrahlten Wärme an die den medizinischen Wärmeübertrager umgebende Luft ab, was sich ungünstig auf die Effizienz der Wärmeübertragung auswirkt.

Es ist bekannt medizinische Wärmeübertrager als Disposable- Heizkassetten (Einmalartikel) herzustellen. Aus Kostengründen werden dafür möglichst kostengünstige Kunststoffe als Werkstoff eingesetzt, die jedoch in der Regel den oben beschriebenen Nachteil einer geringen Wärmeleitfähigkeit aufweisen.

Lokale Temperaturspitzen an dem medizinischen Wärmeübertrager können einerseits die Werkstoffe des medizinischen Wärmeübertragers schädigen oder sogar durch Erweichen, Schmelzen oder Brand zerstören. Andererseits können solche lokalen Temperaturspitzen auch die medizinische Flüssigkeit schädigen, was insbesondere im Hinblick auf die Patientensicherheit zu verhindern ist. Nicht zuletzt kann auch der Emitter für Licht überhitzen.

Bekannte konstruktive Maßnahmen gegen das zumindest lokale Auftreten von unzulässigen Temperaturspitzen ist die Verwendung großer Wärmeübertragerflächen sowie die gezielte Strahlungslenkung und Strahlungsabschirmung jeweils unter Inkaufnahme hoher Kosten und einer verringerten Effizienz der Wärmeübertragung.

Es ist daher eine Aufgabe der vorliegenden Erfindung einen verbesserten medizinischen Wärmeübertrager anzugeben.

**Zusammenfassung**

Diese Aufgabe wird gelöst mit dem Gegenstand des unabhängigen Patentanspruchs 1.

Vorteilhafte Ausführungsformen sind Gegenstände der Unteransprüche. Die Vorteile des medizinischer Wärmeübertragers nach Anspruch 1 können ungeschmälert mit einem medizinischen Schlauchsatz gemäß Anspruch 12 erreicht werden.

Medizinische Flüssigkeiten im Sinne der vorliegenden Lehre schließen beispielsweise Flüssigkeiten aus der Gruppe Blut, Blutplasma, Dialysierflüssigkeit, Substitutionsflüssigkeit, physiologische Kochsalzlösung, Medikamentenlösung, Kontrastmittel, Infusionslösung, Peritonealdialyselösung sowie dem Fachmann bekannte Mischungen solcher Flüssigkeiten ein, sie sind jedoch nicht auf die genannten Beispiele beschränkt. Beispiele für Mischungen solcher Flüssigkeiten sind beispielsweise Blut vermischt mit Substitutionsflüssigkeit und/oder physiologischer Kochsalzlösung, und/oder Medikamentenlösung, und/oder Kontrastmittel und/oder Infusionslösung.

Entsprechend einem ersten Aspekt weist der medizinische Wärmeübertrager mindestens eine Flüssigkeitskammer mit mindestens einer die Flüssigkeitskammer flüssigkeitsdicht ausbildenden Außenwandung auf, wobei die Flüssigkeitskammer konfiguriert ist zum Aufnehmen und/oder Durchleiten der medizinischen Flüssigkeit und wobei die Flüssigkeitskammer mindestens ein zumindest abschnittsweise innerhalb der Flüssigkeitskammer angeordnetes, als Absorber für Licht konfiguriertes Bauteil aufweist, das so angeordnet ist, dass beim Aufnehmen und/oder Durchleiten der medizinischen Flüssigkeit zumindest abschnittsweise ein direkter Kontakt zwischen dem als Absorber für Licht konfigurierten Bauteil und der medizinischen Flüssigkeit vorliegt.

Unter einem als Absorber für Licht konfigurierten Bauteil im Einklang mit der vorliegenden Lehre wird ein Bauteil verstanden, das vorgesehen und geeignet ist, eingestrahltes Licht zu einem überwiegenden Anteil zu absorbieren.

Entsprechend einem weiteren Aspekt weist das als Absorber für Licht konfigurierte Bauteil einen Absorptionsgrad $\alpha_2$ für Licht und einem Transmissionsgrad $\tau_2$ für Licht auf, wobei bevorzugt in dem Wellenlängenbereich des Lichts von 200 nm bis 3.000 nm und besonders bevorzugt in dem Wellenlängenbereich des Lichts von 350 nm bis 2.000 nm der Absorptionsgrad $\alpha_2$ für Licht sehr viel größer ist als der Transmissionsgrad $\tau_2$ für Licht.

Entsprechend einem weiteren Aspekt ist das als Absorber für Licht konfigurierte Bauteil aus einem Werkstoff gefertigt, dessen Absorptionsspektrum bevorzugt in dem Wellenlängenbereich des Lichts von 200 nm bis 3.000 nm und besonders bevorzugt in dem Wellenlängenbereich des Lichts von 350 nm bis 2.000 nm zumindest ein lokales Maximum oder ein absolutes Maximum des Absorptionskoeffizienten aufweist.

Der Wärmeübergang von dem als Absorber für Licht konfigurierten Bauteil auf die medizinische Flüssigkeit bedingt eine ausreichende Wärmeleitung. Wenn eine Strahlung in geringer Eindringtiefe absorbiert wird, dann reicht beispielsweise eine Wärmeleitfähigkeit von Kunststoffen mit beispielsweise 0,15 W/mK aus, um die Wärme an die medizinische Flüssigkeit abzugeben.

Das als Absorber für Licht konfigurierte Bauteil kann entsprechend einem weiteren Aspekt in der Flüssigkeitskammer zum einseitigen oder beidseitigen oder allseitigen direkten Kontakt mit der medizinischen Flüssigkeit angeordnet sein, das heißt der direkte Kontakt kann durch Überströmen oder Umströmen des als Absorber für Licht konfigurierten Bauteils mit der medizinischen Flüssigkeit erfolgen. Besonders vorteilhaft für einen effizienten konvektiven Wärmeübergang ist ein beidseitiges oder allseitiges Umströmen des als Absorber für Licht konfigurierten Bauteils mit der medizinischen Flüssigkeit.

Das als Absorber für Licht konfigurierte Bauteil kann zum Zwecke der Oberflächenvergrößerung eine dreidimensionale Struktur aufweisen um den Wärmeübergang zu verbessern. Beispielsweise kann die dreidimensionale Struktur als Rippen oder Stege ausgeführt sein. Solche Rippen oder Stege des als Absorber für Licht konfigurierten Bauteils können auch dazu vorgesehen werden, um im Zusammenwirken mit entgegengesetzten Rippen oder Stege an der Innenseite der Außenwandung des medizinischen Wärmeübertragers eine mehrfache mäanderförmige Strömungsumlenkung der medizinischen Flüssigkeit zu erzwingen um den Wärmeübergang von dem als Absorber konfigurierten Bauteil auf die medizinische Flüssigkeit zu verbessern. Des Weiteren kann die dreidimensionale Struktur zur Erzeugung einer turbulenten Strömung ausgebildet sein um den Wärmeübergang weiter zu verbessern.

Das als Absorber für Licht konfigurierte Bauteil kann auf seiner Oberfläche dreidimensionale Leitstrukturen zum Erzeugen einer Rotationsströmung aufweisen, so dass das als Absorber für Licht konfigurierte Bauteil von der medizinischen Flüssigkeit beispielsweise schraubenförmig rotierend umströmt wird.

Das als Absorber für Licht konfigurierte Bauteil kann schwarz oder dunkel für sichtbares Licht sein.

Das als Absorber für Licht konfigurierte Bauteil kann durch geeignete Werkstoffauswahl konfiguriert sein zur Absorption von Licht in einem breiten Spektrum der Wellenlänge oder es kann durch geeignete andere Werkstoffauswahl konfiguriert sein zur selektiven Absorption von Licht eines engen Spektrums der Wellenlänge.

Als Werkstoff für das als Absorber für Licht konfigurierte Bauteil kann beispielsweise Keramik oder Metall oder Kunststoff oder ein anderer Werkstoff mit ausreichend hoher Wärmeleitfähigkeit ausgewählt werden. Durch eine ausreichend hohe Wärmeleitfähigkeit wird beim Bestrahlen mit Licht eine im Wesentlichen homogene Temperaturverteilung in dem als Absorber für Licht konfigurierte Bauteil erzielt, so dass Temperaturspitzen vermieden werden und die medizinische Flüssigkeit homogen erwärmt wird.

Das als Absorber für Licht konfigurierte Bauteil kann aus einem compoundierten Werkstoff, insbesondere aus einem compoundierten thermoplastischen spritzgussfähigen Kunststoff oder aus einem compoundierten, thermoplastischen extrusionsfähigen Kunststoff, oder aus einem mittels Metalldampf beschichteten Spritzgussteil oder einem mittels Metalldampf beschichteten Extrusionsteil oder aus einem Metall, insbesondere aus Edelstahl, oder aus einem kerami-

schen Werkstoff, insbesondere $Al_2O_3$ oder AlN gefertigt sein.

Das als Absorber für Licht konfigurierte Bauteil kann aus einem compoundierten thermoplastischen Kunststoff mit metallischen und/oder keramischen und/oder oxydischen Füllmaterialien bestehen oder das als Absorber für Licht konfigurierte Bauteil kann aus einem thermoplastischen Kunststoff mit eingefügten Schichten aus diesen Füllmaterialien bestehen.

Die Flüssigkeitskammer kann entsprechend einem weiteren Aspekt mindestens einen Einlass in der Außenwandung der Flüssigkeitskammer zum Zuführen der medizinischen Flüssigkeit in die Flüssigkeitskammer und/oder mindestens einen Auslass in der Außenwandung der Flüssigkeitskammer zum Abführen der mindestens einen medizinischen Flüssigkeit aus der Flüssigkeitskammer aufweisen.

Entsprechend einem anderen Aspekt kann die Flüssigkeitskammer einen Port und/oder ein Septum zum Zuführen und/oder Entnehmen der medizinischen Flüssigkeit aufweisen.

Die Flüssigkeitskammer kann abschnittsweise oder insgesamt im Wesentlichen starr ausgeführt sein oder aber abschnittsweise oder insgesamt flexibel ausgeführt sein. Die Flüssigkeitskammer kann einteilig gefertigt sein oder aus mindestens zwei Teilen flüssigkeitsdicht zusammengefügt sein. Ein Beispiel für eine abschnittsweise starr ausgeführte Flüssigkeitskammer ist eine durch Spritzgießen oder Thermoformen hergestellte Flüssigkeitskassette mit einem im Wesentlichen starren Hartteil, das Kavitäten und/oder Kanäle zum Führen der medizinischen Flüssigkeit aufweist, wobei die Kavitäten und/oder Kanäle mit einer flexiblen Folie abgedeckt sind und ein Inneres der Flüssigkeitskammer ausbilden und wobei das Hartteil und die flexible Folie zusammen die Außenwandung der Flüssigkeitskammer ausbilden.

Eine beispielhafte insgesamt im Wesentlichen starr ausgeführte Flüssigkeitskammer kann aus zwei Spritzgussteilen zusammengesetzt sein, von denen mindestens eines eine Kavität aufweist, beispielsweise aus einem im Wesentlichen starren ersten Gehäuseteil und einem im Wesentlichen starren zweiten Gehäuseteil, wobei beide Teile flüssigkeitsdicht miteinander verbunden sind oder verbunden werden können, so dass die mindestens eine Kavität ein Inneres der Flüssigkeitskammer ausbildet und das ersten Gehäuseteil zusammen mit dem zweiten Gehäuseteil die Außenwandung der Flüssigkeitskammer ausbildet.

Eine beispielhafte insgesamt flexibel ausgeführte Flüssigkeitskammer ist ein Flüssigkeitsbeutel. Die Außenwandung der Flüssigkeitskammer wird dabei durch zwei flexible Folienabschnitte ausgebildet. Das als Absorber für Licht konfigurierte Bauteil ist in diesem Ausführungsbeispiel eine schwarze Folie, die zumindest abschnittsweise zwischen den beiden flexiblen Folien der Außenwand verschweißt ist.

Entsprechend einem weiteren Aspekt weist der Wärmeübertrager mindestens einen als Lichtdurchlass konfigurierten Abschnitt der Außenwandung der Flüssigkeitskammer auf.

Entsprechend einem weiteren Aspekt ist der Wärmeübertrager einteilig durch Spritzblasen aus wenigstens einem thermoplastischen Kunststoff hergestellt.

Unter einem als Lichtdurchlass konfigurierten Abschnitt der Außenwandung der Flüssigkeitskammer wird im Einklang mit der vorliegenden Lehre ein Bauteil verstanden, das vorgesehen und geeignet ist, eingestrahltes Licht zu einem überwiegenden Anteil durchzulassen und nur einen geringen Teil des eingestrahlten Lichts zu reflektieren oder zu absorbieren. Somit erwärmt sich der als Lichtdurchlass konfigurierte Abschnitt bei Lichteinstrahlung nur geringfügig und eingestrahltes Licht wird mit hoher Effizienz durchgelassen. Dadurch werden Temperaturspitzen an der Außenwandung der Flüssigkeitskammer vermieden, weil sich diese kaum erwärmt.

Entsprechend einem weiteren Aspekt weist der als Lichtdurchlass konfigurierte Abschnitt der Außenwandung der Flüssigkeitskammer einen Absorptionsgrad $\alpha_1$ für Licht und einen Transmissionsgrad $\tau_1$ für Licht auf, wobei bevorzugt in dem Wellenlängenbereich des Lichts von 200 nm bis 3.000 nm und besonders bevorzugt in dem Wellenlängenbereich des Lichts von 350 nm bis 2.000 nm der Transmissionsgrad $\tau_1$ für Licht sehr viel größer ist als der Absorptionsgrad $\alpha_1$ für Licht.

Entsprechend einem weiteren Aspekt ist der als Lichtdurchlass konfigurierte Abschnitt der Außenwandung der Flüssigkeitskammer aus einem Werkstoff gefertigt, dessen Transmissionsspektrum bevorzugt in dem Wellenlängenbereich des Lichts von 200 nm bis 3.000 nm und besonders bevorzugt in dem Wellenlängenbereich des Lichts von 350 nm bis 2.000 nm zumindest ein lokales Maximum oder ein absolutes Maximum des Transmissionskoeffizienten aufweist.

Der als Lichtdurchlass konfigurierte Abschnitt der Außenwandung der Flüssigkeitskammer kann für sichtbares Licht zumindest transluzent oder sogar transparent ausgeführt sein.

Der als Lichtdurchlass konfigurierte Abschnitt der Außenwandung kann sich über einen Teil der Außenwandung oder im Wesentlichen über die gesamte Außenwandung erstrecken. Dadurch werden vorteilhaft Temperaturspitzen aufgrund der Lichteinstrahlung an der Außenwandung der Flüssigkeitskammer vermieden.

Der als Lichtdurchlass konfigurierte Abschnitt der Außenwandung kann aus einer Öffnung mit einem umlaufenden flüssigkeitsdichten Rahmen bestehen, in den eine transparente Scheibe eingesetzt ist. Die Scheibe kann beispielsweise aus Glas, Quarzglas oder aus Polyacryl bestehen. Die Scheibe kann im Wesentlichen rund oder im Wesentlichen rechteckig sein.

Entsprechend einem weiteren Aspekt kann der medizinische Wärmeübertrager Bestandteil eines medizinischen Schlauchsatzes sein, wobei der medizinische Schlauchsatz zusätzlich weitere dem Fachmann bekannte Komponenten aufweisen kann.

Entsprechend einem weiteren Aspekt kann der medizinische Wärmeübertrager integrierter Bestandteil einer medizinischen Flüssigkeitsbehandlungsvorrichtung sein, wobei die Flüssigkeitsbehandlungsvorrichtung zumindest einen Emitter für Licht zur Beheizung des medizinischen Wärmeübertragers aufweist.

Ein Beispiel für eine solche Ausführungsform ist eine Hämodialysemaschine mit einem Dialsysierflüssigkeitskreislauf und einer Quelle für Substitutionsflüssigkeit, wobei die Dialysierflüssigkeit und/oder die Substitutionsflüssigkeit mittels eines in der Dialysemaschine installierten, offenbarungsgemäßen medizinischen Wärmeübertragers erwärmt wird. Bei einer solchen Ausführungsform wird der medizinischen Wärmeübertrager typischerweise von einem qualifizierten Servicetechniker ausgetauscht und ist für den längerfristigen Gebrauch bestimmt.

Entsprechend einem weiteren Aspekt kann der medizinischer Wärmeübertrager zur Ankopplung an eine medizinische Flüssigkeitsbehandlungsvorrichtung konfiguriert sein, wobei die medizinische Flüssigkeitsbehandlungsvorrichtung zumindest einen Emitter für Licht und zumindest eine als Lichtdurchlass konfigurierten Ankoppelfläche für den medizinischen Wärmeübertrager aufweist.

Ein Beispiel einer solchen Ausführungsform ist eine Hämodialysemaschine, bei der das Blut in einem extrakorporalen Blutkreislauf mittels eines Wärmeübertragers nach der vorliegenden Offenbarung erwärmt werden soll. Bei einer solchen Ausführungsform wird der medizinischen Wärmeübertrager vom Anwender der Hämodialysemaschine vor Beginn jeder Blutbehandlung jeweils als neues Disposable an die Ankoppelfläche der Hämodialysemaschine angekoppelt.

Entsprechend einem weiteren Aspekt weist die medizinische Flüssigkeitsbehandlungsvorrichtung eine Steuerungs- und/oder Regelungseinheit mit Controller konfiguriert zum Steuern- und/oder oder Regeln der Strahlungsleistung des mindestens einen Emitters für Licht auf. Die Steuerungs- und/oder Regelungseinheit mit Controller kann in Signalverbindung stehen mit einer Temperaturmessvorrichtung in oder an der Flüssigkeitsleitung, vorzugsweise am Flüssigkeitsauslass des medizinischen Wärmeübertragers. Die Temperaturmessvorrichtung kann Bestandteil der Flüssigkeitsbehandlungsvorrichtung sein, wobei die Messstelle der Flüssigkeitsleitung in eine entsprechende Messaufnahme an der Flüssigkeitsbehandlungsvorrichtung eingelegt wird.

Der Emitter für Licht kann konfiguriert sein zum Emittieren von Licht in einem breiten Spektrum der Wellenlänge, beispielsweise umfassend das sichtbare Licht oder umfassend das sichtbare Licht und das Infrarotlicht.

Alternativ kann der Emitter für Licht konfiguriert sein zum selektiven Emittieren von Licht in einem engen Spektrum der Wellenlänge.

Besonders vorteilhaft ist der Emitter für Licht konfiguriert zum selektiven Emittieren eines Spektrums von Licht in dem die Wellenlängen kürzer sind als die Wellenlängen von Infrarotlicht.

Es ist in einer Ausführungsform möglich, den Emitter für Licht als Emitter nur für Infrarotlicht auszugestalten. Das als Absorber für Licht konfigurierte Bauteil ist bei einer solchen Ausführungsform durch Wahl des Werkstoffs konfiguriert zur selektiven Absorption von Infrarotlicht.

Der Leistungsbereich der elektrischen Leistungsaufnahme des Emitters für Licht liegt bevorzugt im Bereich von 1 W bis 1.000 W und besonders bevorzugt im Bereich von 100 W bis 600 W.

**Kurzbeschreibung der Figuren**

Im Folgenden werden Ausführungsbeispiele des Wärmeübertragers nach der vorliegenden Lehre unter Bezugnahme auf die Figuren näher erläutert. Anhand der in den Figuren dargestellten Ausführungsbeispiele werden weitere Einzelheiten und Vorteile näher beschrieben. Die Bezugszeichen in den Figuren haben jeweils durchgängig in allen Figuren die gleiche Bedeutung.

Es zeigen:

**Fig. 1** eine schematische Schnitt-Darstellung der Anordnung eines Wärmeübertragers an einem Emitter für Licht
**Fig. 2** eine schematische Darstellung eines medizinischen Schlauchsatzes mit einem integrierten medizinischen Wärmeübertrager
**Fig. 3** eine schematische Darstellung einer medizinischen Flüssigkeitsbehandlungsvorrichtung mit einer Ankoppelfläche zur Ankopplung eines medizinischen Wärmeübertragers

**Detaillierte Beschreibung der Figuren**

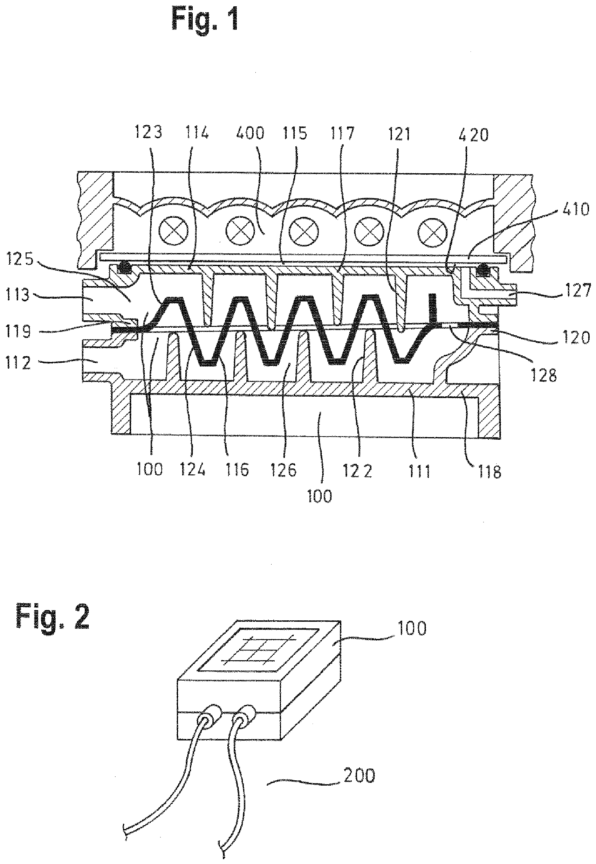

Die Figur 1 zeigt eine schematische Schnitt-Darstellung der Anordnung eines medizinischen Wärmeübertragers 100 an einem Emitter für Licht 400. Der Emitter für Licht 400 ist in diesem Ausführungsbeispiel eine maschinenseitige Komponente einer medizinischen Flüssigkeitsbehandlungsvorrichtung und der medizinische Wärmeübertrager 100 ist als Disposable ausgestaltet.

Der Emitter für Licht 400 weist mehrere in der Figur 1 durch Kreuze symbolisierte Glühbirnen auf, die nebeneinander angeordnet sind, um den Lichtdurchlass 410 des Emitters für Licht 400 möglichst gleichmäßig zu bestrahlen. Der Lichtdurchlass 410 des Emitters für Licht 400 ist im vorliegenden Ausführungsbeispiel eine ebene, transparente Glasscheibe aus farblosem Glas. Die den Glühbirnen abgewandte Seite des Lichtdurchlasses 410 bildet eine Ankoppelfläche 420 die durch ihre Form und ihre Maße konfiguriert ist zum Ankoppeln des medizinischen Wärmeübertragers 100. Nicht in Figur 1 dargestellt sind Vorrichtungen zum Fixieren des Wärmeübertragers 100 an der Ankoppelfläche 420. Dafür geeignet sind beispielsweise Klemmvorrichtungen oder Türen zum Anpressen und Halten des medizinischen Wärmeübertragers.

Der medizinische Wärmeübertrager 100 ist im vorliegenden Ausführungsbeispiel aus einem ersten Gehäuseteil 117 mit einem ersten umlaufenden Flansch 119 und einem zweiten Gehäuseteil 118 mit einem zweiten umlaufenden Flansch 120 aufgebaut, wobei beide Gehäuseteile schalenförmig ausgestaltet sind. Der ersten Gehäuseteil 117 ist mit seinem ersten umlaufenden Flansch 119 mit dem zweiten umlaufenden Flansch 120 des zweiten Gehäuseteils 118 flüssigkeitsdicht verbunden, wobei als Dichtung der umlaufende Rand des Absorbers für Licht 116 zwischen den Flanschen angeordnet ist. Der Absorber für Licht 116 ist somit zwischen dem Gehäuseteil 117 und einem zweiten Gehäuseteil 118 eingespannt und unterteilt des Innere der Flüssigkeitskammer 110 in einen ersten Strömungskanal 125 der dem Lichtdurchlass der Flüssigkeitskammer 114 zugewandt ist und in einen zweiten Strömungskanal 126, der dem Lichtdurchlass der Flüssigkeitskammer 114 abgewandt ist. Der zweite Strömungskanal 126 weist einen Einlass der Außenwandung 112 zum Zuführen der medizinischen Flüssigkeit in den zweiten Strömungskanal 126 auf. Der erste Strömungskanal 125 weist einen Auslass der Außenwandung 113 zum Abführen der medizinischen Flüssigkeit aus dem ersten Strömungskanal 125 auf. Im vorliegenden Ausführungsbeispiel liegen der Einlass und der Auslass nahe beieinander, so dass die Strömungskanäle 125, 126 in Reihe geschaltet insgesamt im gezeigten Querschnitt eine U-förmige Flüssigkeitskammer ausbilden, wobei die Strömungskanäle 125, 126 über eine flüssigkeitsdurchlässige Öffnung 128 in dem Absorber für Licht 116 in Verbindung stehen.

Wenn der medizinische Wärmeübertrager des vorliegenden Ausführungsbeispiels von der medizinischen Flüssigkeit durchströmt wird, dann erwärmt sich die medizinischen Flüssigkeit zuerst in dem kälteren zweiten Strömungskanal 126, der dem Lichtdurchlass der Flüssigkeitskammer 114 abgewandt ist und danach in dem wärmeren ersten Strömungskanal 125 der dem Lichtdurchlass der Flüssigkeitskammer 114 zugewandt ist. Dadurch wird eine besonders effiziente Wärmeübertragung ermöglicht.

Der erste Gehäuseteil 117 ist in einem Abschnitt als Lichtdurchlass der Flüssigkeitskammer 114 ausgebildet. Der Werkstoff der Außenwandung ist zumindest in diesem Bereich eben und transparent ausgeführt und des Weiteren durch Form und Maße konfiguriert zum Ankoppeln des medizinischen Wärmeübertragers 100 an die Ankoppelfläche des Emitters für Licht 420, so dass Licht vom Lichtdurchlass des Emitters für Licht 410 in den Lichtdurchlass der Flüssigkeitskammer 114 einstrahlen kann.

Im vorliegenden Ausführungsbeispiel weist der medizinische Wärmeübertrager an der Außenseite des zweiten Gehäuseteils 118 eine Versteifung in Form eines umfangenden Stegs auf, so dass der medizinische Wärmeübertrager insgesamt eine hohe Steifigkeit aufweist. Im vorliegenden Ausführungsbeispiel kann über einen Absaugkanal 127 Luft aus dem Zwischenraum zwischen dem Lichtdurchlass der Flüssigkeitskammer 114 und dem Lichtdurchlass des Emitters für Licht 410 abgesaugt werden, um die Absorption von Licht in dem Zwischenraum zu minimieren und eine besonders sichere Ankopplung des Wärmeübertragers zu gewährleisten. In anderen Ausführungsformen kann jedoch auf den Absaugkanal 127 verzichtet werden.

Der Lichtdurchlass des Emitters für Licht 410 und der Lichtdurchlass der Flüssigkeitskammer 114 absorbieren bei Lichteinstrahlung nur einen kleinen Teil der eingestrahlten Leistung und lassen den überwiegenden Teil der eingestrahlten Leistung in die Flüssigkeitskammer 110 einstrahlen. In der Flüssigkeitskammer wird der überwiegende Teil der eingestrahlten Leistung von dem Absorber für Licht 116 absorbiert, wodurch sich der Absorber für Licht 116 erwärmt.

Wenn der medizinische Wärmeübertrager an den Emitter für Licht 400 angekoppelt ist und von medizinischer Flüssigkeit durchströmt wird und der in Betrieb befindliche Emitter für Licht 400 Licht emittiert, so gibt der Absorber für Licht seine absorbierte Wärme durch Wärmeleitung und Abstrahlung von Infrarotstrahlung an die medizinische Flüssigkeit ab, wodurch sie die medizinische Flüssigkeit schonend erwärmt.

Figur 2 zeigt eine Ausgestaltung des Wärmeübertragers aus Figur 1, wobei der Wärmeübertrager 100 in einen medizinischen Schlauchsatz 200 integriert ist. Der medizinische Schlauchsatz ist in Figur 2 lediglich schematisch durch eine zuführende Leitung und eine abführende Leitung an dem medizinischen Wärmeübertrager 100 angedeutet. Für den Fachmann ist offensichtlich, dass der medizinische Schlauchsatz 200 außer dem medizinischen Wärmeübertrager und den genannten Schlauchleitungen zusätzlich bekannte weitere Komponenten aufweisen kann. Solche bekannten weiteren Komponenten können beispielsweise die üblichen Komponenten eines Blutschlauchsatzes für die extrakorporale Blutbehandlung oder eines medizinischen Schlauchsatzes aus dem Gebiet der Infusionstechnik sein, wie beispielsweise Konnektoren, Pumpschlauchsegment zum Anschluss einer Rollenpumpe, Dialysator, Luftblasenabscheider, Tropfkammer, Gerinnselfänger, Schlauchklemmen, Messküvetten, Zuspritzstellen.

Der medizinische Schlauchsatz 200 kann im Fertigungsprozess gemeinsam mit dem verbundenen medizinischen Wärmeübertrager sterilisiert und verpackt werden, was einen Handhabungsvorteil im klinischen Einsatz darstellt.

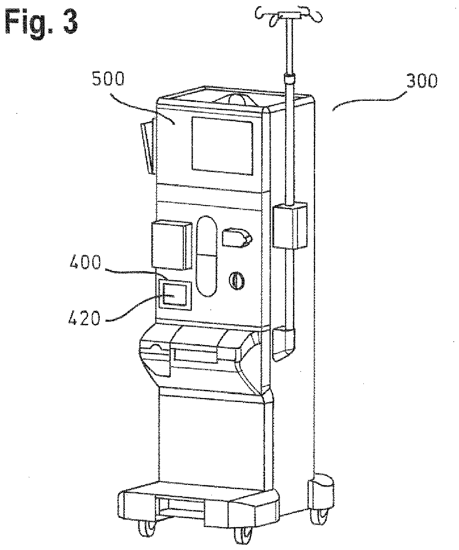

Figur 3 zeigt eine schematische Darstellung einer medizinischen Flüssigkeitsbehandlungsmaschine 300 mit einem Emitter für Licht 400 und einer Ankoppelfläche des Emitters für Licht 420, die konfiguriert ist zum Ankoppeln eines Wärmeübertragers 100 als Komponente des medizinischen Schlauchsatzes 200 aus Figur 2. Nicht dargestellt ist die bei medizinischen Flüssigkeitshandlungsmaschinen zum Fördern der medizinischen Flüssigkeit in dem medizinischen Schlauchsatz 200 bekannten Vorrichtungen, im vorliegenden Ausführungsbeispiel eine Rollenpumpe (in Figur 3 nicht dargestellt), in die das Pumpschlauchsegment des medizinischen Schlauchsatzes eingelegt wird. Außerdem nicht dargestellt ist die bei medizinischen Flüssigkeitshandlungsmaschinen bekannte Vorrichtung zum Messen der Temperatur an einem medizinischen Schlauchsatz 200, im vorliegenden Ausführungsbeispiel als Messaufnahme für ein Schlauchsegment des medizinischen Schlauchsatzes ausgeführt und angeordnet stromabwärts vom Auslass des medizinischen Wärmeübertragers 100.

Die medizinische Flüssigkeitsbehandlungsmaschine 300 weist eine Steuerungs- und/oder Regelungseinheit 500 mit Controller auf, die in Signalverbindung mit der Vorrichtung zum Messen der Temperatur an dem medizinischen Schlauchsatz 200 stromabwärts des medizinischen Wärmeübertragers 100 steht. Außerdem steht die Steuerungs- und/oder Regelungseinheit 500 in Signalverbindung mit dem Emitter für Licht. Des Weiteren weist die Steuerungs- und/oder Regelungseinheit 500 eine Eingabeeinheit zum manuellen Eingeben eines numerischen Werts einer Solltemperatur auf.

Im Controller der Steuerungs- und/oder Regelungseinheit 500 ist ein Computerprogramm mit Programmcode abgespeichert, das die Leistung des Emitters für Licht regelt, so dass die vorgegebenen Solltemperatur innerhalb vorgegebener Grenzen eingehalten wird, wenn der Programmcode in der Steuerungs- und/oder Regelungseinheit 500 abläuft.

**Bezugszeichenliste**

| Bezugszeichen | Bezeichnung |
|---|---|
| 100 | Medizinischer Wärmeübertrager |
| 110 | Flüssigkeitskammer |
| 111 | Außenwandung |
| 112 | Einlass der Außenwandung |
| 113 | Auslass der Außenwandung |
| 114 | Lichtdurchlass der Flüssigkeitskammer |
| 115 | Ankoppelfläche des Wärmeübertragers |
| 116 | Absorber für Licht |
| 117 | erster Gehäusesteil |
| 118 | zweiter Gehäuseteil |
| 119 | erster umlaufender Flansch |
| 120 | zweiter umlaufender Flansch |
| 121 | Dreidimensionale Struktur des ersten Gehäuseteils |
| 122 | Dreidimensionale Struktur des zweiten Gehäuseteils |
| 123 | erste dreidimensionale Fläche des Absorbers |
| 124 | zweite dreidimensionale Fläche des Absorbers |
| 125 | erster Strömungskanal |
| 126 | zweiter Strömungskanal |
| 127 | Absaugkanal |
| 128 | flüssigkeitsdurchlässige Öffnung |
| 200 | medizinischer Schlauchsatz |
| 300 | medizinische Flüssigkeitsbehandlungsvorrichtung |
| 400 | Emitter für Licht |

(fortgesetzt)

| Bezugszeichen | Bezeichnung |
|---|---|
| 410 | Lichtdurchlass |
| 420 | Ankoppelfläche |
| 500 | Steuerungs- und/oder Regelungseinheit |

**Patentansprüche**

1. Medizinischer Wärmeübertrager (100) zum Erwärmen einer medizinischen Flüssigkeit mittels Licht, aufweisend mindestens eine Flüssigkeitskammer (110) konfiguriert zum Aufnehmen und/oder Durchleiten der medizinischen Flüssigkeit mit mindestens einer die Flüssigkeitskammer (110) flüssigkeitsdicht ausbildenden Außenwandung (111) mindestens einen Einlass (112) in der Außenwandung (111) der Flüssigkeitskammer (110) zum Zuführen der medizinischen Flüssigkeit in die Flüssigkeitskammer (110) und mindestens einen Auslass (113) in der Außenwandung (111) der Flüssigkeitskammer (110) zum Abführen der medizinischen Flüssigkeit aus der Flüssigkeitskammer (110) mindestens einen als Lichtdurchlass (114) konfigurierten Abschnitt der Außenwandung (111) der Flüssigkeitskammer (110)
**dadurch gekennzeichnet, dass**
der medizinische Wärmeübertrager (100) mindestens ein innerhalb der Flüssigkeitskammer (110) angeordnetes, als Absorber (116) für Licht konfiguriertes Bauteil aufweist, das so angeordnet ist, dass beim Aufnehmen und/oder Durchleiten der medizinischen Flüssigkeit für einen effizienten konvektiven Wärmeübergang beidseitig oder allseitig ein direkter Kontakt zwischen dem als Absorber (116) für Licht konfigurierten Bauteil und der medizinischen Flüssigkeit vorliegt,
wobei das als Absorber (116) für Licht konfigurierte Bauteil zum Zwecke der Oberflächenvergrößerung eine dreidimensionale Struktur aufweist um den Wärmeübergang zu verbessern und
die dreidimensionale Struktur zur Erzeugung einer turbulenten Strömung ausgebildet ist, und
die dreidimensionale Struktur als Rippen oder Stege ausgeführt ist, um im Zusammenwirken mit entgegengesetzten Rippen oder Stegen an der Innenseite der Außenwandung (111) des medizinischen Wärmeübertragers eine mehrfache mäanderförmige Strömungsumlenkung der medizinischen Flüssigkeit zu erzwingen um den Wärmeübergang von dem als Absorber konfigurierten Bauteil auf die medizinische Flüssigkeit zu verbessern,
wobei das als Absorber (116) für Licht konfigurierte Bauteil vorgesehen und geeignet ist, eingestrahltes Licht zu einem überwiegenden Anteil zu absorbieren, wobei das als Absorber (116) für Licht konfigurierte Bauteil aus einem Werkstoff gefertigt ist, dessen Absorptionsspektrum in dem Wellenlängenbereich des Lichts von 200 nm bis 3.000 nm zumindest ein lokales Maximum oder ein absolutes Maximum des Absorptionskoeffizienten aufweist.

2. Medizinischer Wärmeübertrager (100) gemäß Anspruch 1, wobei der als Lichtdurchlass (114) konfigurierte Abschnitt der Außenwandung (111) der Flüssigkeitskammer (110) im Wesentlichen eine ebene Fläche oder eine gewölbte Fläche bildet.

3. Medizinischer Wärmeübertrager (100) gemäß Anspruch 1 oder 2, wobei der medizinischer Wärmeübertrager (100) zumindest abschnittsweise als lichtdurchlässiges Kunststoff-Spritzgussteil ausgeführt ist.

4. Medizinischer Wärmeübertrager (100) gemäß einem der Ansprüche 1 bis 3, wobei zumindest der als Lichtdurchlass (114) konfigurierte Abschnitt der Außenwandung (111) der Flüssigkeitskammer (110) transparent ist.

5. Medizinischer Wärmeübertrager (100) gemäß einem der Ansprüche 1 bis 4, wobei der medizinische Wärmeübertrager (100) aus einem ersten Gehäuseteil (117) mit einem ersten umlaufenden Flansch (119) und einem zweiten Gehäuseteil (118) mit einem zweiten umlaufenden Flansch (120) besteht, wobei der erste umlaufende Flansch (119) mit dem zweiten umlaufende Flansch (120) flüssigkeitsdicht verbunden ist, wobei das als Absorber (116) für Licht konfigurierte Bauteil zwischen dem ersten umlaufenden Flansch (119) und dem zweiten umlaufenden Flansch (120) eingefasst ist und sich in die Flüssigkeitskammer (110) hinein erstreckt.

6. Medizinischer Wärmeübertrager (100) gemäß Anspruch 5, wobei zumindest der erste Gehäuseteil (117) und/oder der zweite Gehäuseteil (118) auf der dem Inneren der Flüssigkeitskammer (110) zugewandten Oberfläche zumindest abschnittsweise eine dreidimensionale Struktur (121, 122) aufweist, wobei das als Absorber (116) für Licht konfigurierte Bauteil mit einer ersten Oberfläche eine im Wesentlichen geschlossene erste dreidimensionale Fläche (123) bildet und/oder mit einer zweiten Oberfläche eine im Wesentlichen geschlossene zweite dreidimensionale Fläche (124) bildet.

**7.** Medizinischer Wärmeübertrager (100) gemäß einem der Ansprüche 5 bis 6, wobei die dem Inneren der Flüssigkeitskammer (110) zugewandten Oberfläche des ersten Gehäuseteils (117) mit einer ersten Oberfläche des als Absorber (116) für Licht konfigurierten Bauteils einen ersten Strömungskanal (125) für die mindestens eine medizinische Flüssigkeit ausbildet und die dem Inneren der Flüssigkeitskammer (110) zugewandten Oberfläche des zweiten Gehäuseteils (118) mit einer zweiten Oberfläche des als Absorber (116) für Licht konfigurierten Bauteils einen zweiten Strömungskanal (126) für die mindestens eine medizinische Flüssigkeit ausbildet wobei der erste Strömungskanal (125) flüssigkeitsführend in den zweiten Strömungskanal (126) übergeht, wobei der erste Strömungskanal (125) den mindestens einen Einlass (112) zum Zuführen der mindestens einen medizinischen Flüssigkeit aufweist und der zweite Strömungskanal (126) den mindestens einen Auslass (113) zum Abführen der mindestens einen medizinischen Flüssigkeit aufweist.

**8.** Medizinischer Wärmeübertrager (100) gemäß Anspruch 7, **dadurch gekennzeichnet, dass**
der erste Strömungskanal (125) durch die im Wesentlichen geschlossenen dreidimensionalen Flächen der dem Inneren der Flüssigkeitskammer (110) zugewandten Oberfläche des ersten Gehäuseteils (117) und der ersten Oberfläche des als Absorber (116) für Licht konfigurierten Bauteils mäanderförmig ausgestaltet ist
und/oder
der zweite Strömungskanal (126) durch die im Wesentlichen geschlossenen dreidimensionalen Flächen der dem Inneren der Flüssigkeitskammer (110) zugewandten Oberfläche des zweiten Gehäuseteils (118) und der zweiten Oberfläche des als Absorber (116) für Licht konfigurierten Bauteils mäanderförmig ausgestaltet ist.

**9.** Medizinischer Wärmeübertrager (100) gemäß einem der vorhergehenden Ansprüche, wobei das als Absorber (116) für Licht konfigurierte Bauteil aus einem compoundierten Werkstoff, insbesondere aus einem compoundierten thermoplastischen spritzgussfähigen Kunststoff oder aus einem compoundierten, thermoplastischen extrusionsfähigen Kunststoff, oder aus einem mittels Metalldampf beschichteten Spritzgussteil oder einem mittels Metalldampf beschichteten Extrusionsteil oder aus einem Metall, insbesondere aus Edelstahl, oder aus einem keramischen Werkstoff, insbesondere $Al_2O_3$ oder AIN gefertigt ist.

**10.** Medizinischer Wärmeübertrager (100) gemäß einem der vorhergehenden Ansprüche wobei der als Lichtdurchlass (114) konfigurierte Abschnitt der Außenwandung (111) der Flüssigkeitskammer (110) aus einem Werkstoff aus der Gruppe Glas, Quarzglas, Polypropylen (PP), Polycarbonat (PC) gefertigt ist.

**11.** Medizinischer Wärmeübertrager (100) gemäß einem der Ansprüche 1 bis 10, wobei der medizinische Wärmeübertrager (100) als medizinischer Einmalartikel, insbesondere als medizinische Flüssigkeitskassette ausgeführt ist.

**12.** Medizinischer Schlauchsatz (200) aufweisend mindestens einen medizinischen Wärmeübertrager (100) gemäß einem der Ansprüche 1 bis 11, wobei der mindestens eine Einlass (112) in der Außenwandung (111) der Flüssigkeitskammer (110) zum Zuführen der mindestens einen medizinischen Flüssigkeit in die Flüssigkeitskammer (110) mit einer Schlauchleitung des medizinischen Schlauchsatzes (200) verbunden ist und/oder der mindestens eine Auslass (113) in der Außenwandung (111) der Flüssigkeitskammer (110) zum Abführen der mindestens einen medizinischen Flüssigkeit aus der Flüssigkeitskammer (110) mit einer Schlauchleitung des medizinischen Schlauchsatzes verbunden ist.

**Claims**

**1.** A medical heat exchanger (100) for heating a medical liquid by means of light, having
at least one liquid chamber (110) configured to receive and/or conduct the medical liquid having at least one outer wall (111) forming the liquid chamber (110) in a liquid-tight manner
at least one inlet (112) in the outer wall (111) of the liquid chamber (110) for supplying the medical liquid into the liquid chamber (110) and at least one outlet (113) in the outer wall (111) of the liquid chamber (110) for removing the medical liquid from the liquid chamber (110)
at least one portion of the outer wall (111) of the liquid chamber (110) configured as a light aperture (114)
**characterized in that**
the medical heat exchanger (100) has at least one component, arranged inside the liquid chamber (110), configured as an absorber (116) for light, which is arranged so that on receiving and/or conducting the medical liquid, for an efficient convective heat transfer on both sides or on all sides, a direct contact is present between the component configured as absorber (116) for light and the medical liquid,

wherein the component configured as absorber (116) for light has a three-dimensional structure for the purpose of enlarging the surface in order to improve the heat transfer and

the three-dimensional structure is formed for generating a turbulent flow, and

the three-dimensional structure is embodied as ribs or webs, in order to impose a multiple meandering flow deflection of the medical liquid in cooperation with opposed ribs or webs on the inner side of the outer wall (111) of the medical heat exchanger, in order to improve the heat transfer from the component, configured as absorber, to the medical liquid,

wherein the component configured as absorber (116) for light is provided and suited to absorb irradiated light to a predominant proportion, wherein the component configured as absorber (116) for light is made from a material, the absorption spectrum of which in the wavelength range of the light from 200 nm to 3,000 nm has at least a local maximum or an absolute maximum of the absorption coefficient.

2. The medical heat exchanger (100) according to Claim 1, wherein the portion of the outer wall (111) of the liquid chamber (110) configured as light aperture (114) forms substantially a flat surface or a curved surface.

3. The medical heat exchanger (100) according to Claim 1 or 2, wherein the medical heat exchanger (100) is embodied at least partially as a transparent plastic injection moulded part.

4. The medical heat exchanger (100) according to one of Claims 1 to 3, wherein at least the portion of the outer wall (111) of the liquid chamber (110) configured as light aperture (114) is transparent.

5. The medical heat exchanger (100) according to one of Claims 1 to 4, wherein the medical heat exchanger (100) consists of a first housing part (117) with a first circumferential flange (119) and a second housing part (118) with a second circumferential flange (120), wherein the first circumferential flange (119) is connected with the second circumferential flange (120) in a liquid-tight manner, wherein the component configured as absorber (116) for light is enclosed between the first circumferential flange (119) and the second circumferential flange (120) and extends into the liquid chamber (110).

6. The medical heat exchanger (100) according to Claim 5, wherein at least the first housing part (117) and/or the second housing part (118) on the surface facing the interior of the liquid chamber (110) has at least partially a three-dimensional structure (121, 122), wherein the component configured as absorber (116) for light forms with a first surface a substantially closed first three-dimensional area (123) and/or forms with a second surface a substantially closed second three-dimensional area (124).

7. The medical heat exchanger (100) according to one of Claims 5 to 6, wherein the surface of the first housing part (117), facing the interior of the liquid chamber (110), forms with a first surface of the component configured as absorber (116) for light a first flow channel (125) for the at least one medical liquid, and the surface of the second housing part (118), facing the interior of the liquid chamber (110), forms with a second surface of the component configured as absorber (116) for light a second flow channel (126) for the at least one medical liquid, wherein the first flow channel (125) continues, in a liquid-directing manner into the second flow channel (126), wherein the first flow channel (125) has the at least one inlet (112) for supplying the at least one medical liquid, and the second flow channel (126) has the at least one outlet (113) for removing the at least one medical liquid.

8. The medical heat exchanger (100) according to Claim 7, **characterized in that**
the first flow channel (125) through the substantially closed three-dimensional areas of the surface of the first housing part (117) facing the interior of the liquid chamber (110), and of the first surface of the component configured as absorber (116) for light, is configured in a meandering manner
and/or
the second flow channel (126) through the substantially closed three-dimensional areas of the surface of the second housing part (118) facing the interior of the liquid chamber (110), and of the second surface of the component configured as absorber (116) for light, is configured in meandering manner.

9. The medical heat exchanger (100) according to one of the preceding claims, wherein the component configured as absorber (116) for light is made from a compounded material, in particular from a compounded thermoplastic plastic able to be injection moulded, or from a compounded thermoplastic plastic able to be extruded, or from an injection moulded part coated by means of metal vapour, or from an extrusion part coated by means of metal vapour, or from a metal, in particular from high-grade steel, or from a ceramic material, in particular Al$_2$O$_3$ or AlN.

10. The medical heat exchanger (100) according to one of the preceding claims, wherein the portion of the outer wall (111) of the liquid chamber (110) configured as light aperture (114) is made from a material of the group: glass, quartz glass, polypropylene (PP), polycarbonate (PC).

11. The medical heat exchanger (100) according to one of Claims 1 to 10, wherein the medical heat exchanger (100) is embodied as a disposable medical article, in particular as a medical liquid cassette.

12. A medical tubing set (200), having at least one medical head exchanger (100) according to one of Claims 1 to 11, wherein the at least one inlet (112) in the outer wall (111) of the liquid chamber (110) for supplying the at least one medical liquid into the liquid chamber (110) is connected with a tubing line of the medical tubing set (200) and/or the at least one outlet (113) in the outer wall (111) of the liquid chamber (110) for removing the at least one medical liquid from the liquid chamber (110) is connected with a tubing line of the medical tubing set.


**Revendications**

1. Échangeur thermique médical (100) servant à réchauffer un liquide médical au moyen de lumière, présentant
   au moins une chambre à liquide (110) configurée pour recevoir et/ou conduire le liquide médical de manière étanche au liquide et comportant au moins une paroi extérieure (111) constituant la chambre à liquide (110).
   au moins une admission (112) dans la paroi extérieure (111) de la chambre à liquide (110) pour acheminer le liquide médical dans la chambre à liquide (110) et au moins une évacuation (113) dans la paroi extérieure (111) de la chambre à liquide (110) pour évacuer le liquide médical de la chambre à liquide (110),
   au moins une section configurée sous forme de passage de lumière (114) de la paroi extérieure 111) de la chambre à liquide (110),
   **caractérisé en ce que**
   l'échangeur thermique médical (100) présente au moins une pièce disposée à l'intérieur de la chambre à liquide (110) et configurée sous forme d'un absorbeur (116) de lumière, qui est disposée de manière à ce que, lors de la réception et/ou de la conduite du liquide médical, il y ait, pour un échange thermique convectif efficace des deux côtés ou de tous côtés, un contact direct entre la pièce configurée sous forme d'un absorbeur (116) de lumière et le liquide médical,
   la pièce configurée sous forme d'un absorbeur (116) de lumière présentant, aux fins de l'agrandissement de la surface, une structure tridimensionnelle afin d'améliorer le transfert thermique et
   la structure tridimensionnelle étant configurée pour générer un écoulement turbulent, et
   la structure tridimensionnelle étant configurée sous forme de nervures ou de traverses pour, en interaction avec des nervures ou traverses opposées, au niveau de la face intérieure de la paroi extérieure (111) de l'échangeur thermique, imposer une dérivation multiple d'écoulement en forme de méandres du liquide médical afin d'améliorer le transfert thermique de la pièce configurée sous forme d'un absorbeur sur le liquide médical,
   la pièce configurée sous forme d'un absorbeur (116) de lumière étant prévue pour et apte à absorber de la lumière irradiée dans sa grande majorité, la pièce configurée sous forme d'un absorbeur (116) de lumière étant fabriquée dans un matériau dont le spectre d'absorption présente, dans la plage de longueur d'onde de la lumière de 200 nm à 3000 nm, au moins un maximum local ou un maximum absolu du coefficient d'absorption.

2. Échangeur thermique médical (100) selon la revendication 1, dans lequel la section configurée sous forme d'un passage de lumière (114) de la paroi extérieure (111) de la chambre à liquide (110) constitue substantiellement une surface plane ou une surface bombée.

3. Échangeur thermique médical (100) selon la revendication 1 ou 2, dans lequel l'échangeur thermique médical (100) est réalisé au moins par sections sous forme d'une pièce en plastique moulée par injection et transparente.

4. Échangeur thermique médical (100) selon une des revendications 1 à 3, dans lequel au moins la section configurée sous forme d'un passage lumineux (114) de la paroi extérieure (111) de la chambre à liquide (110) est transparente.

5. Échangeur thermique médical (100) selon une des revendications 1 à 4, dans lequel l'échangeur thermique médical (100) est composé d'une première pièce de boîtier (117) dotée d'une première bride périphérique (119) et d'une seconde pièce de boîtier (118) dotée d'une seconde bride périphérique (120), la première bride périphérique (119) étant connectée de manière étanche au liquide à la seconde bride périphérique (120), la pièce configurée sous forme d'un absorbeur (116) de lumière étant sertie entre la première bride périphérique (119) et la seconde bride périphérique (120) et s'étendant vers l'intérieur de la chambre à liquide (110).

6. Échangeur thermique médical (100) selon la revendication 5, dans lequel au moins la première pièce de boîtier (117) et/ou la seconde pièce de boîtier (118) présente, sur sa surface tournée vers l'intérieur de la chambre à liquide (110), au moins par sections, une structure tridimensionnelle (121, 122), la pièce réalisée sous forme d'un absorbeur (116) de lumière constituant avec une première surface une première surface tridimensionnelle substantiellement fermée (123) et/ou avec une seconde surface une seconde surface tridimensionnelle sensiblement fermée (124)

7. Échangeur thermique médical (100) selon une des revendications 5 à 6, dans lequel la surface tournée vers l'intérieur de la chambre à liquide (110) de la première pièce de boîtier (117) constitue, avec une première surface de la pièce configurée sous forme d'un absorbeur (116) de lumière, un premier canal d'écoulement (125) pour l'au moins un liquide médical et la surface tournée vers l'intérieur de la chambre à liquide (110) de la seconde pièce de boîtier (118) constituant, avec une seconde surface de la pièce configurée sous forme d'un absorbeur (116) de lumière, un second canal d'écoulement (126) pour l'au moins un liquide médical, le premier canal d'écoulement (125) transitant en conduisant le liquide dans le second canal d'écoulement (126), le premier canal d'écoulement (125) présentant l'au moins une admission (112) pour l'acheminement de l'au moins un liquide médical et le second canal d'écoulement (126) présentant l'au moins une évacuation (113) pour l'évacuation de l'au moins un liquide médical.

8. Échangeur thermique médical (100) selon la revendication 7, **caractérisé en ce que**
le premier canal d'écoulement (125) est constitué en forme de méandres par les surfaces tridimensionnelles substantiellement fermées de la surface tournée vers l'intérieur de la chambre à liquide (110) de la première pièce de boîtier (117) et la première surface de la pièce configurée sous forme d'un absorbeur (116) de lumière
et/ou
le second canal d'écoulement (126) est constitué par les surfaces tridimensionnelles substantiellement fermées de la surface tournée vers l'intérieur de la chambre à liquide (110) de la seconde pièce de boîtier (118) et la seconde surface de la pièce configurée sous forme d'un absorbeur (116) de lumière.

9. Échangeur thermique médical (100) selon une des revendications précédentes, dans lequel la pièce configurée sous forme d'un absorbeur (116) de lumière est composée d'un matériau composite, en particulier d'une matière thermoplastique composée pouvant être moulée par injection ou d'une matière thermoplastique composée pouvant être extrudée, ou d'une pièce moulée par injection revêtue par vaporisation métallique ou d'une pièce extrudée revêtue par vaporisation métallique ou d'un métal, en particulier de l'acier inoxydable, ou d'un matériau céramique, en particulier de l'$Al_2O_3$ ou de l'AIN.

10. Échangeur thermique médical (100) selon une des revendications précédentes, dans lequel la section configurée sous forme d'un passage de lumière (114) de la paroi extérieure (111) de la chambre à liquide (110) est composée d'un matériau faisant partie du groupe composé du verre, du verre de quartz, du polypropylène (PP), du polycarbonate (PC).

11. Échangeur thermique médical (100) selon une des revendications 1 à 10, dans lequel l'échangeur thermique médical (100) est réalisé sous forme d'un article médical à usage unique, en particulier d'une cartouche de liquide médical.

12. Jeu de tuyaux médicaux (200), présentant au moins un échangeur thermique médical (100) selon une des revendications 1 à 11, dans lequel l'au moins une admission (112) de la paroi extérieure (111) de la chambre à liquide (110), pour l'acheminement de l'au moins un liquide médical dans la chambre à liquide (110), est connectée à une canalisation du jeu de tuyaux médicaux (200) et/ou l'au moins une évacuation (113) de la paroi extérieure (111) de la chambre à liquide (110), pour l'évacuation de l'au moins un liquide médical de la chambre à liquide (110), est connectée à une canalisation du jeu de tuyaux médicaux.

# Fig. 1

# Fig. 2

# Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20030135250 A1
- US 20090299271 A1
- DE 102010002895 A1

- EP 2311514 A2
- WO 03061740 A1